# FASCICULE DE BREVET EUROPEEN

(11) **EP 1 762 172 B1**
(45) Date de publication et mention de la délivrance du brevet: **04.05.2011**
(21) Numéro de dépôt: 06018884.4
(22) Date de dépôt: 08.09.2006
(51) Int. Cl.: A61B 1/12, A61B 1/015, B08B 9/02

(54) **Dispositif et procédé pour le nettoyage d'un endoscope**
Vorrichtung und Verfahren zur Reinigung eines Endoskops
Device and method for cleaning an endoscope

(30) Priorité: 09.09.2005 NL 1029911
(43) Date de publication de la demande: 14.03.2007
(73) Titulaire: Lancer Industrie, 31170 Tournefeuille (FR)
(72) Inventeur: Rietveld, Karel Nicolaas, 6659 KC Wamel (NL)
(74) Mandataire: Cabinet BARRE LAFORGUE & associés

(56) Documents cités:
- EP-A- 1 433 412
- WO-A-93/17727
- DE-A1- 3 414 963
- FR-A- 2 722 123
- US-A- 4 637 378

## Description

L'invention concerne un dispositif pour le nettoyage à l'aide d'un liquide de rinçage d'un canal eau/air et d'un canal d'aspiration d'un endoscope, plus particulièrement d'un endoscope souple, qui est pourvu d'une chambre eau/air cylindrique, pourvue d'une première bride sur laquelle et dans laquelle est disposé, dans l'état d'utilisation, un premier organe de commande et d'une chambre d'aspiration cylindrique, pourvue d'une deuxième bride, sur laquelle et dans laquelle est disposé, dans l'état d'utilisation, un deuxième organe de commande, le dispositif étant constitué par une plaque de montage pourvue de premiers moyens d'étanchéité pour la chambre eau/air et de deuxièmes moyens d'étanchéité pour la chambre d'aspiration.

Un dispositif de ce type est connu. Après l'utilisation d'un endoscope, par exemple lors de l'inspection d'un creux corporel d'un patient, la bride de fixation est écartée, puis les organes de commande peuvent être enlevés, puis le dispositif est mis en place et est fixé avec une bride de fixation pouvant être déplacée. Ensuite, on fait passer un liquide de nettoyage dans l'endoscope, qui nettoie et désinfecte le canal eau/air, le canal d'aspiration, la chambre eau/air et la chambre d'aspiration. Le dispositif connu est en fait constitué par deux organes en forme de tige, chacun pourvu d'un joint annulaire, qui sont poussés dans la chambre eau/air et la chambre d'aspiration. L'inconvénient réside ici en ce que les pièces de la chambre eau/air et de la chambre d'aspiration, qui se trouvent au-dessus des joints annulaires pendant le nettoyage, ne sont pas nettoyées ni désinfectées.

On connaît des documents US 4,637,378 et WO93/1772 des dispositifs de nettoyage d'endoscopes permettant un nettoyage complet des chambres. Cependant les systèmes de verrouillage permettant la mise en place de ces dispositifs sont complexes et peu commodes à utiliser, demandant l'usage des deux mains pour les fixer.

Le dispositif selon l'invention résout cet inconvénient et est caractérisé en ce que la plaque de montage est pourvue d'un premier joint annulaire qui repose pendant le nettoyage sur la première bride et d'un deuxième joint annulaire qui repose pendant le nettoyage sur la deuxième bride, suite à quoi la partie interne de la chambre eau/air et la partie interne de la chambre d'aspiration peuvent être nettoyées et désinfectées complètement.

Une forme de réalisation avantageuse du dispositif selon l'invention est caractérisée en ce que la chambre eau/air est pourvue d'une alimentation en eau, d'une évacuation d'eau, d'une alimentation en air et d'une évacuation d'air et que le premier couvercle est pourvu d'un manche avec un aspirateur qui y est monté, réalisé de telle manière que l'aspirateur sépare, pendant le nettoyage, l'alimentation et l'évacuation d'eau de l'alimentation et de l'évacuation d'air, de telle manière que celles-ci peuvent être nettoyées et désinfectées séparément les unes des autres. De préférence, l'aspirateur est pourvu d'un troisième joint annulaire, qui repose pendant le nettoyage contre une face interne de la chambre eau/air, de telle manière qu'une fuite le long de l'aspirateur peut être négligée.

Selon un autre aspect de l'invention, une forme de réalisation avantageuse est caractérisée en ce que la chambre d'aspiration est pourvue d'une entrée d'aspiration et d'une sortie d'aspiration et le deuxième couvercle est pourvu de moyens de guidage destinés à guider vers le couvercle, pendant le nettoyage, un liquide de rinçage, de telle manière qu'un flux suffisant est généré, même au niveau de la paroi de la chambre à proximité du couvercle, pour garantir un nettoyage complet et une désinfection complète.

Une autre forme de réalisation avantageuse et facile à réaliser est caractérisée en ce que les moyens de guidage comprennent un organe en forme de tuyau, pourvu d'orifices au niveau de la première extrémité, à proximité du deuxième couvercle, suite à quoi une partie du liquide de nettoyage qui s'écoule s'écoulera dans ces orifices le long du couvercle. De préférence, l'organe en forme de tuyau est pourvu d'une deuxième extrémité élargie, pour favoriser un flux de liquide de nettoyage via les orifices.

Une autre forme de réalisation avantageuse est caractérisée en ce que la deuxième extrémité élargie est pourvue d'un quatrième joint annulaire. D'une part, on peut ainsi réaliser une résistance à l'écoulement relativement élevée, tandis qu'il reste possible, d'autre part, de glisser l'organe en forme de tuyau dans la chambre d'aspiration sans abîmer la paroi de la chambre d'aspiration. Il y a toutefois alors lieu de veiller à choisir le diamètre de la deuxième extrémité élargie de telle manière que du liquide de rinçage puisse passer le long de la deuxième extrémité élargie et le quatrième joint annulaire.

Une autre forme de réalisation avantageuse qui permet de simplifier de manière importante la mise en place du dispositif est caractérisée en ce que la plaque de montage est fixée dans et sur un organe en forme de bol, pourvu de deux proéminences présentant une section tranversale quasiment en forme de L, qui peuvent être vissées et serrées avant le nettoyage, en vue de fixer l'organe en forme de bol, sur l'endoscope sous la première et la deuxième bride.

L'invention concerne également un procédé pour le nettoyage d'un canal eau/air et d'un canal d'aspiration d'un endoscope et en particulier d'un endoscope souple, un premier organe de commande étant enlevé d'une chambre eau/air et un deuxième organe de commande étant enlevé d'une chambre d'aspiration, les deux chambres étant ensuite fermées et un liquide de nettoyage et désinfectant étant guidé via une pièce de raccordement dans le canal eau/air, le canal d'aspiration, la chambre eau/air et la chambre d'aspiration. Le procédé selon l'invention est caractérisé en ce qu'on utilise lors du nettoyage un dispositif selon l'une quelconque des revendications 1 à 9.

L'invention est explicitée à l'aide des figures suivantes, dans lesquelles :
la Figure 1A représente de manière schématique, en vue latérale, un endoscope souple ;
la Figure 1B représente, de manière schématique, l'extrémité d'un endoscope ;
la Figure 2A représente schématiquement en coupe transversale un dispositif selon l'état de la technique ;
la Figure 2B représente schématiquement en vue de face ce dispositif avec un capot de fixation ;
la Figure 3A représente schématiquement, en coupe transversale, une forme de réalisation possible d'un dispositif selon l'invention ;
la Figure 3B représente schématiquement cette forme de réalisation en vue de face.

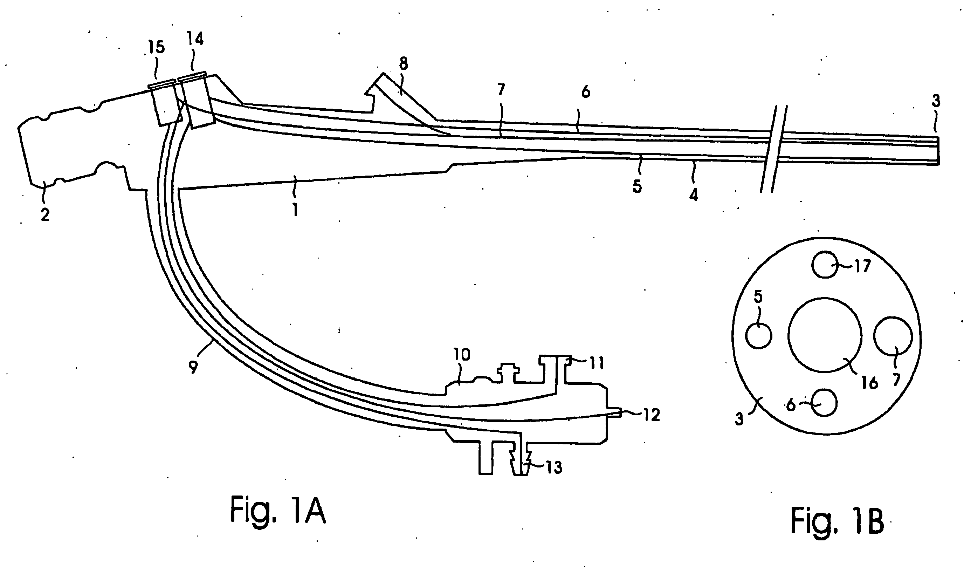

La Figure 1A représente schématiquement, en vue latérale, un endoscope souple, constitué par une poignée 1 pourvue d'un viseur 2 avec lequel on peut par exemple regarder dans un creux corporel d'un patient à l'aide d'un assemblage en fibre ou d'un raccordement électrique non représenté via l'extrémité 3 de l'endoscope qui est introduit dans le creux corporel. L'assemblage en fibre passe dans un flexible 4 long, souple, qui simplifie l'introduction. Le flexible 4 comporte encore une conduite 5 qui permet d'acheminer de l'eau vers l'extrémité 3, une conduite 6 via laquelle de l'air peut être acheminé vers l'extrémité 3 et un canal d'évacuation 7 pour l'eau et/ou l'air, et des instruments pouvant en outre être introduits via une porte 8, par exemple pour le prélèvement de biopsies ou pour cautériser un vaisseau sanguin. De plus, le flexible 4 comporte généralement encore une fibre non représentée, qui permet d'introduire de la lumière dans le creux corporel. L'endoscope est en outre raccordé via un deuxième flexible 9 à un bloc de couplage 10 avec un raccord 11 pour l'eau, un raccord 12 pour l'air et un raccord 13 pour l'évacuation de l'eau et/ou de l'air aspiré via le canal d'évacuation 7. Dans la poignée 1, on a agencé une chambre eau/air 14, dans laquelle on place, dans l'état d'utilisation, un organe de commande avec lequel on peut, selon les souhaits, acheminer de l'eau et/ou de l'air vers l'extrémité 3. En outre, on a agencé dans la poignée 1 une chambre d'aspiration 15 dans laquelle on place, dans l'état d'utilisation, un organe de commande avec lequel on peut aspirer, selon les souhaits, de l'eau et/ou de l'air via l'extrémité 3. Dans la Figure 1A, on représente une situation peu avant le nettoyage de l'endoscope, un liquide de nettoyage étant acheminé via les raccords 11, 12, 13 qui doit nettoyer et désinfecter la chambre eau/air 14, la chambre d'aspiration 15 et les conduites 4, 5, 6. Il est dans tous les cas clair que les faces supérieures ouvertes de la chambre eau/air 14 et de la chambre d'aspiration 15 doivent être fermées, par exemple avec un couvercle. Ceci n'est toutefois pas suffisant, car on ne peut ainsi pas garantir que tant la conduite 5 que la conduite 6 soient rincées suffisamment. Pour cette raison, on place dans la chambre eau/air 14 également un aspirateur non représenté dans cette figure, qui sépare la conduite 5 et la conduite 6 l'une de l'autre.

La Figure 1B représente schématiquement l'extrémité d'un endoscope, avec au centre une lentille 16 pour former l'image qu'on peut voir dans le viseur 2, une conduite 5 pour l'eau, une conduite 6 pour l'air, une conduite d'aspiration 7 ainsi qu'une fibre 17 pour la lumière.

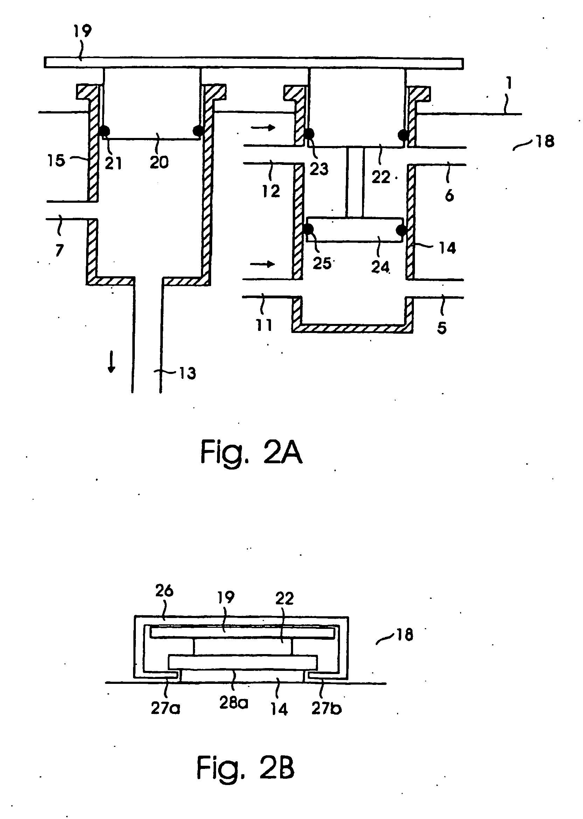

La Figure 2A représente schématiquement, en coupe transversale, un dispositif 18 selon l'état de la technique. Dans la poignée 1, on a disposé la chambre eau/air 14 et la chambre d'aspiration 15, dont les faces supérieures doivent être fermées lors du rinçage. Le dispositif 18 est constitué par une plaque de montage 19 sur laquelle est fixé un aspirateur 20, pourvu d'un joint annulaire 21 qui ferme la chambre d'aspiration 15 ainsi qu'un aspirateur 22 pourvu d'un joint annulaire 23 qui ferme la chambre eau/air 14. Sur l'aspirateur 22 on a placé un aspirateur 24, pourvu d'un joint annulaire 25 qui sépare la chambre eau/air 14 en deux moitiés, de telle manière que le liquide de rinçage alimenté via l'entrée 11 s'écoule vers la conduite 5 et le liquide de rinçage alimenté via l'entrée 12 s'écoule vers la conduite 6. L'inconvénient réside en ce que la partie de la chambre d'aspiration 15 qui se trouve au-dessus du joint annulaire 21 n'est pas rincée alors que la partie qui se trouve juste au-dessous du joint annulaire 21 sera rincée insuffisamment. De plus, la partie de la chambre eau/air 14 qui se trouve au-dessus du joint annulaire 23 ne sera pas rincée non plus.

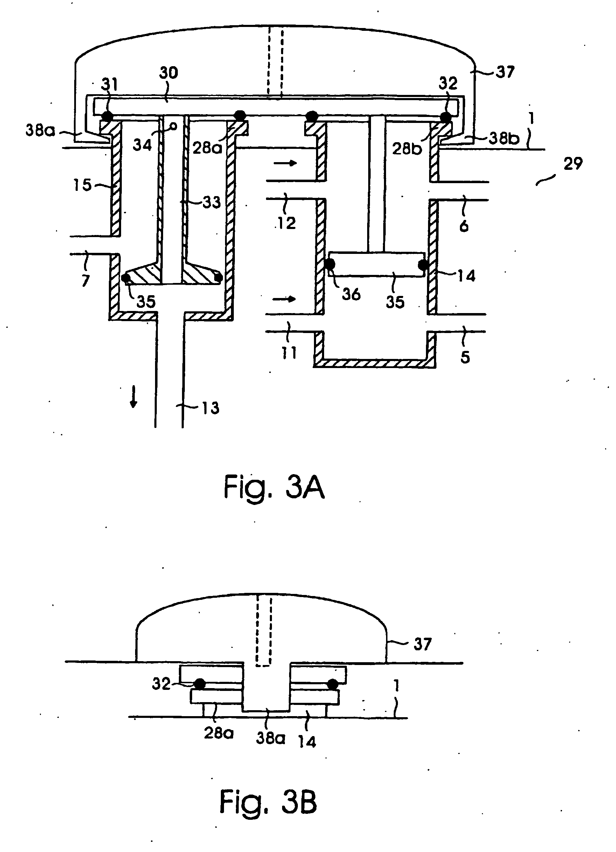

La Figure 2B représente schématiquement, en vue de face, ce dispositif 18 avec un capot de fixation 26. Le dispositif 18 est mis en place comme indiqué dans la Figure 3A, suite à quoi le capot de fixation 26 est glissé dans la plaque de châssis 19, les deux bords en forme de L 27a, 27b glissant sous l'extrémité en forme de bride 28a de la chambre d'aspiration 14 et, non visible dans la figure, sous l'extrémité en forme de bride 28b de la chambre eau/air 15.

La Figure 3A représente schématiquement, en coupe transversale, une forme de réalisation possible d'un dispositif 29 selon l'invention. Dans la poignée 1, on a disposé la chambre eau/air 14 et la chambre d'aspiration 15, dont les faces supérieures doivent être fermées lors du rinçage. Le dispositif 29 est constitué par une plaque de montage 30 dans laquelle est agencé un joint annulaire 31 qui se place de manière étanche, dans la position montrée, contre l'extrémité en forme de bride de la chambre d'aspiration 15 et dans laquelle un joint annulaire 32 est également mis en place qui, dans la position représentée, se place de manière étanche contre l'extrémité en forme de bride de la chambre eau/air 14. Sur la plaque de montage 30 on a également fixé un organe 33 en forme de tuyau, pourvu de trous 34 et s'élargissant sur la face inférieure et pourvu d'un joint annulaire 35 qui ne se place pas contre la paroi de la chambre, de telle manière qu'une petite quantité de liquide de rinçage peut passer. La plus grande partie du liquide de rinçage qui entre via la conduite d'aspiration 7 s'écoule ainsi de manière forcée, via l'organe en forme de tuyau 33 et les trous 34, vers l'évacuation 13. Sur la plaque de montage 30 on a également placé un aspirateur 35, pourvu d'un joint annulaire 36 qui sépare la chambre eau/air 14 en deux moitiés, de telle manière que le liquide de rinçage alimenté via l'entrée 11 s'écoule vers la conduite 5 et le liquide de rinçage alimenté via l'entrée 12 s'écoule vers la conduite 6. Un capot 37 est fixé de manière à pouvoir tourner sur la plaque de montage 30, dont deux bords 38a, 38b présentant une section en forme de L peuvent être tournés sous les extrémités 28a, 28b en forme de bride, ce qui permet d'obtenir un verrouillage de type baïonnette qui enfonce les joints annulaires 31, 32 et qui permet d'obtenir une bonne étanchéité en un tour de main.

La Figure 3B représente schématiquement, en vue de face, cette forme de réalisation, avec le capot 37 et le bord 38a qui est tourné sous l'extrémité 28a en forme de bride.

## Revendications

1. Dispositif pour le nettoyage à l'aide d'un liquide de rinçage d'un canal eau/air et d'un canal d'aspiration d'un endoscope, plus particulièrement d'un endoscope souple, qui est pourvu d'une chambre eau/air (14) cylindrique, pourvue d'une première bride sur laquelle et dans laquelle est disposé, dans l'état d'utilisation, un premier organe de commande et d'une chambre d'aspiration (15) cylindrique, pourvue d'une deuxième bride, sur laquelle et dans laquelle est disposé, dans l'état d'utilisation, un deuxième organe de commande, le dispositif étant constitué par une plaque de montage (30) pourvue de premiers moyens d'étanchéité pour la chambre eau/air (14) et de deuxièmes moyens d'étanchéité pour la chambre d'aspiration (15) **caractérisé en ce que** la plaque de montage (30) est pourvue d'un premier joint annulaire (32) qui repose, pendant le nettoyage, sur la première bride (28b) et d'un deuxième joint annulaire (31), qui repose, pendant le nettoyage, sur la deuxième bride (28a) et **en ce que** la plaque de montage (30) est fixée dans et sur un capot (37) tournant sur la plaque, pourvu de deux proéminences (38a, 38b) présentant une section transversale quasiment en forme de L qui peuvent être vissées avant le nettoyage, en vue de la fixation du capot (37), sur l'endoscope sous la première bride (28b) et la deuxième bride (28a).

2. Dispositif selon la revendication 1, **caractérisé en ce que** la chambre eau/air (14) est pourvue d'une alimentation en eau (11), d'une évacuation d'eau (5), d'une alimentation en air (12) et d'une évacuation d'air (6) et les premiers moyens d'étanchéité sont pourvus d'un manche sur lequel est monté un aspirateur (35), réalisé de telle manière que l'aspirateur, pendant le nettoyage, sépare l'alimentation en eau et l'évacuation d'eau de l'alimentation en air et de l'évacuation de l'air.

3. Dispositif selon la revendication 2, **caractérisé en ce que** l'aspirateur (35) est pourvu d'un troisième joint annulaire (36), qui repose, pendant le nettoyage, contre une face intérieure de la chambre eau/air.

4. Dispositif selon l'une quelconque des revendications précédentes, **caractérisé en ce que** la chambre d'aspiration (15) est pourvue d'une entrée d'aspiration (7) et d'une sortie d'aspiration (13) et les deuxièmes moyens d'étanchéité sont pourvus de moyens de guidage (33, 34, 35) pour guider pendant le nettoyage le liquide de rinçage vers lesdits deuxièmes moyens d'étanchéité.

5. Dispositif selon la revendication 4, **caractérisé en ce que** les moyens de guidage comprennent un organe en forme de tuyau (33), pourvu d'orifices (34) au niveau de la première extrémité, à proximité des deuxièmes moyens d'étanchéité.

6. Dispositif selon la revendication 5, **caractérisé en ce que** l'organe en forme de tuyau (33) est pourvu d'une deuxième extrémité élargie.

7. Dispositif selon la revendication 6, **caractérisé en ce que** la deuxième extrémité élargie est pourvue d'un quatrième joint annulaire (35).

8. Dispositif selon la revendication 7, **caractérisé en ce que** le diamètre de la deuxième extrémité élargie est choisi de telle manière que du liquide de rinçage peut passer le long de la deuxième extrémité élargie et du quatrième joint annulaire.

9. Procédé pour le nettoyage d'un canal eau/air et d'un canal d'aspiration d'un endoscope et en particulier d'un endoscope souple, un premier organe de commande étant enlevé d'une chambre eau/air et un deuxième organe de commande étant enlevé d'une chambre d'aspiration, les deux chambres étant ensuite fermées et un liquide de nettoyage et désinfectant étant guidé via une pièce de raccordement dans le canal eau/air, le canal d'aspiration, la chambre eau/air et la chambre d'aspiration, **caractérisé en ce qu'**on utilise lors du nettoyage un dispositif selon l'une quelconque des revendications 1 à 8.

## Claims

1. A device for cleaning by way of a rinsing liquid a water/air channel and a suction channel of an endoscope, more particularly of a flexible endoscope, which is provided with a cylindrical water/air chamber (14), provided with a first flange on which and in which a first control member is arranged when in use, and a cylindrical suction chamber (15), provided with a second flange on which and in which a second control member is arranged when in use, the device consisting of a mounting plate (30) provided with first sealing means for the water/air chamber (14) and with second sealing means for the suction chamber (15), **characterised in that** the mounting plate (30) is provided with a first annular gasket (32) which rests, during cleaning, on the first flange (28b) and a second annular gasket (31) which rests, during cleaning, on the second flange (28a) and **in that** the mounting plate (30) is fixed in and on a cap (37) which turns on the plate, said cap being provided with two projections (38a, 38b) having a cross-section virtually in the form of an L which may be screwed onto the endoscope under the first flange (28b) and the second flange (28a) prior to cleaning, with the aim of fixing the cap (37) in place.

2. A device according to claim 1, **characterised in that** the water/air chamber (14) is provided with a water feed (11), a water outlet (5), an air feed (12) and an air outlet (6) and the first sealing means are provided with a shaft on which is mounted an suction means (35), embodied in such a way that, during cleaning, the suction means separates the water feed and the water outlet from the air feed and the air outlet.

3. A device according to claim 2, **characterised in that** the suction means (35) is provided with a third annular gasket (36), which rests, during cleaning, against an internal face of the water/air chamber.

4. A device according to any one of the preceding claims, **characterised in that** the suction chamber (15) is provided with a suction inlet (7) and a suction outlet (13) and the second sealing means are provided with guide means (33, 34, 35) for guiding the rinsing liquid towards said second sealing means during cleaning.

5. A device according to claim 4, **characterised in that** the guide means comprise a member in the form of a tube (33), provided with orifices (34) at the level of the first end, in the vicinity of the second sealing means.

6. A device according to claim 5, **characterised in that** the member in the form of a tube (33) is provided with a second, widened end.

7. A device according to claim 6, **characterised in that** the second, widened end is provided with a fourth annular gasket (35).

8. A device according to claim 7, **characterised in that** the diameter of the second, widened end is selected such that rinsing liquid may pass along the second, widened end and the fourth annular gasket.

9. A method of cleaning a water/air channel and a suction channel of an endoscope and in particular of a flexible endoscope, a first control member being removed from a water/air chamber and a second control member being removed from a suction chamber, the two chambers then being closed and a cleaning and disinfectant liquid being guided via a connecting element into the water/air channel, the suction channel, the water/air chamber and the suction chamber, **characterised in that** a device according to any one of claims 1 to 8 is used at the time of cleaning.

## Patentansprüche

1. Vorrichtung für die Reinigung, mithilfe einer Spülflüssigkeit, eines Wasser-/Luftkanals und eines Saugkanals eines Endoskops, insbesondere eines flexiblen Endoskops, das eine erste zylindrische Wasser-/Luftkammer (14) aufweist, mit einem ersten Flansch, an dem und in dem im Gebrauchszustand ein erstes Steuerorgan angeordnet ist, sowie eine zylindrische Saugkammer (15), mit einem zweiten Flansch, an dem und in dem im Gebrauchszustand ein zweites Steuerorgan angeordnet ist, wobei die Vorrichtung aus einer Montageplatte (30) mit ersten Dichtungsmitteln für die Wasser-/Luftkammer (14) und mit zweiten Dichtungsmittel für die Saugkammer (15) besteht, **dadurch gekennzeichnet, dass** die Montageplatte (30) einen ersten Dichtring (32) aufweist, der während der Reinigung auf dem ersten Flansch (28b) ruht, sowie einen zweiten Dichtring (31), der während der Reinigung auf dem zweiten Flansch (28a) ruht, und dass die Montageplatte (30) in und an einer auf der Platte drehbaren Haube (37) befestigt ist, die zwei Überstände (38a, 38b) mit einem etwa L-förmigem Querschnitt aufweist, die vor der Reinigung aufgeschraubt werden können, um die Haube (37) am Endoskop unter dem ersten Flansch (28b) und dem zweiten Flansch (28a) zu befestigen.

2. Vorrichtung gemäß Anspruch 1, **dadurch gekennzeichnet, dass** die Wasser-/Luftkammer (14) eine Wasserversorgung (11), eine Wasserableitung (5), eine Luftversorgung (12) und eine Luftableitung (6) aufweist, und die ersten Dichtungsmittel ein Heft aufweisen, an dem ein Sauger (35) derart montiert ist, dass der Sauger während der Reinigung die Wasserversorgung und die Wasserableitung von der Luftversorgung und der Luftableitung trennt.

3. Vorrichtung gemäß Anspruch 2, **dadurch gekennzeichnet, dass** der Sauger (35) einen dritten Dichtring (36) aufweist, der während der Reinigung an einer Innenseite der Wasser-/Luftkammer anliegt.

4. Vorrichtung gemäß einem der vorherigen Ansprüche, **dadurch gekennzeichnet, dass** die Saugkammer (15) einen Saugeingang (7) und einen Saugausgang (13) aufweist, und dass die zweiten Dichtungsmittel Führungsmittel (33, 34, 35) aufweisen, um die Spülflüssigkeit während der Reinigung zu den genannten zweiten Dichtungsmitteln hin zu leiten.

5. Vorrichtung gemäß Anspruch 4, **dadurch gekennzeichnet, dass** die Führungsmittel ein schlauchförmiges Organ (33) umfassen, das im Bereich des ersten Endes, in der Nähe der zweiten Dichtungsmittel Öffnungen (34) aufweist.

6. Vorrichtung gemäß Anspruch 5, **dadurch gekennzeichnet, dass** das schlauchförmige Organ (33) ein zweites erweitertes Ende aufweist.

7. Vorrichtung gemäß Anspruch 6, **dadurch gekennzeichnet, dass** das zweite erweiterte Ende einen vierten Dichtring (35) aufweist.

8. Vorrichtung gemäß Anspruch 7, **dadurch gekennzeichnet, dass** der Durchmesser des zweiten erweiterten Endes so gewählt wird, dass Spülflüssigkeit entlang dem zweiten erweiterten Ende und dem vierten Dichtring fließen kann.

9. Verfahren für die Reinigung eines Wasser-/Luftkanals und eines Saugkanals eines Endoskops und insbesondere eines flexiblen Endoskops, wobei ein erstes Steuerorgan einer Wasser-/Luftkammer entnommen wird und ein zweites Steuerorgan einer Saugkammer entnommen wird, wobei die beiden Kammern anschließend geschlossen werden, und eine Reinigungs- und Desinfektionsflüssigkeit über ein Anschlussteil in den Wasser-/Luftkanal, den Saugkanal, die Wasser-/Luftkammer und die Saugkammer geführt wird, **dadurch gekennzeichnet, dass** man während der Reinigung eine Vorrichtung gemäß einem der Ansprüche 1 bis 8 verwendet.
